# EUROPEAN PATENT SPECIFICATION

(11) **EP 1 797 885 B1**
(45) Date of publication and mention of the grant of the patent: **26.03.2008**
(21) Application number: 05256202.2
(22) Date of filing: 04.10.2005
(51) Int. Cl.: A61K 31/55, A61K 31/4422

(54) **Stable combinations of amlodipine besylate and benazepril hydrochloride**
Stabile Arzneimittelkombinationen von Amlodipinbesylat und Benazeprilhydrochlorid
Combinations stables d'amlodipine besylate et de benazeprile hydrochloride

(30) Priority: 28.09.2005 US 238496; 28.09.2005 WO PCT/US2005/034930
(43) Date of publication of application: 20.06.2007
(60) Divisional application: 08002835.0
(73) Proprietor: TEVA PHARMACEUTICAL INDUSTRIES LTD., 49131 Petah Tiqva (IL)
(72) Inventor: Kadosh, Mali, Kfar Saba (IL); Leska, Fanny, Herzlia (IL)
(74) Representative: Gallagher, Kirk James

(56) References cited:
- WO-A-02/49645
- WO-A-02/49646
- US-A1- 2002 176 889
- NAIDU ET AL: "Stability indicating RP-HPLC method for simultaneous determination of amlodipine and benazepril hydrochloride from their combination drug product" JOURNAL OF PHARMACEUTICAL AND BIOMEDICAL ANALYSIS, NEW YORK, NY, US, vol. 39, no. 1-2, 1 September 2005 (2005-09-01), pages 147-155, XP005013202 ISSN: 0731-7085

## Description

### FIELD OF INVENTION

The present invention relates to the field of combination therapy of amlodipine with benazepril.

### BACKGROUND OF THE INVENTION

In the fight against cardiovascular disease, our current arsenal of weapons includes angiotensin converting enzyme inhibitors (ACEI) and calcium channel blockers (CCB). Combination therapy of an ACEI with a CCB can achieve synergistic results effective in treating a variety of conditions including hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, stroke, and headache. The combination of the ACEI benazepril and the CCB amlodipine is described by U.S. Patent No. 6,162,802 ("the '802 patent").

Benazepril can be administered as benazepril hydrochloride, which is chemically identified as 3-[[1-(ethoxycarbonyl)-3-phenyl-(1S)-propyl]amino]-2,3,4,5-tetrahydro-2-oxo-1*H*-1-(3S)-benzazepine-1-acetic acid monohydrochloride (C₂₄H₂₈N₂O₅ ·HCl). Amlodipine can be administered as amlodipine besylate, which is chemically identified as (R.S.) 3-ethyl-5-methyl-2-(2-aminoethoxymethyl)-4-(2-chlorophenyl)-1,4-dihydro-6-methyl-3,5-pyridinedicarboxylate benzenesulphonate (C₂₀H₂₅ClN₂O₅.AC₆H₆O₃S). A combination of benazepril hydrochloride and amlodipine besylate is marketed in the United States by Novartis under the trade name LOTREL® as capsules for oral administration. The capsules are formulated in four different strengths with an amount of amlodipine besylate equivalent to 2.5 mg, 5 mg, or 10 mg of amlodipine free base, and 10 mg or 20 mg of benazepril hydrochloride providing for the following available combinations: 2.5/10 mg, 5/10 mg, 5/20 mg, and 10/20 mg. LOTREL® is indicated for the treatment of hypertension.

The '802 patent teaches that "[b]enazepril and amlodipine are physically incompatible substances. Hence, if incorporated into a single dosage form they must be kept physically separated." Col. 3, lines 48-50.

The present inventors have surprisingly discovered a method for making a pharmaceutical composition containing both amlodipine and benazepril wherein physical separation of the two drug components is not required. Such a pharmaceutical composition and methods for making it are provided herein.

### SUMMARY OF THE INVENTION

In one embodiment, the present invention provides a stable pharmaceutical composition comprising benazepril, solvates, esters, or pharmaceutically acceptable salts thereof, and amlodipine or solvates, esters, or pharmaceutically acceptable salts thereof. The pharmaceutical composition is stable, that is, after storage for about 3 months at about 40°C at about 75% relative humidity, it contains no more than 2.5% of (s,s)-diacid by weight relative to the benazepril and/or no more than 0.3% of impurity D by weight relative to the amlodipine. Preferably, the benazepril is benazepril hydrochloride, and the amlodipine is amlodipine besylate.

In a preferred embodiment, the benazepril and the amlodipine are in physical contact with one another.

In another embodiment, the benazepril hydrochloride is prepared by wet granulation, and the amlodipine besylate is prepared by dry processing.

In another embodiment, the present invention provides a method including the steps of: preparing a benazepril composition by wet granulation; preparing an amlodipine composition by dry processing; and contacting the benazepril composition and the amlodipine composition to obtain a combination pharmaceutical composition.

Wet granulation preferably includes the steps of combining benazepril hydrochloride with one or more pharmaceutical excipients preferably selected from the group consisting of pregelatinized starch, lactose monohydrate, starch, crospovidone, and microcrystalline cellulose; adding a granulation solution to obtain a wet granulate, wherein the granulation solution comprises water and optionally, polysorbate 80, povidone, or both; drying the wet granulate to obtain a dry granulate; and milling the dry granulate.

Dry processing preferably includes the steps of sieving a mixture of amlodipine besylate with one or more pharmaceutical excipients preferably selected from the group consisting of calcium phosphate dibasic, sodium starch glycolate, microcrystalline cellulose, and colloidal silicon dioxide; and blending the mixture.

In one embodiment, dry processing further includes sieving one or more additional pharmaceutical excipients, preferably crospovidone and/or magnesium stearate, and blending the additional pharmaceutical excipient with the mixture.

In another embodiment, the method includes a step of encapsulating the combination pharmaceutical composition.

### BRIEF DESCRIPTION OF THE FIGURES

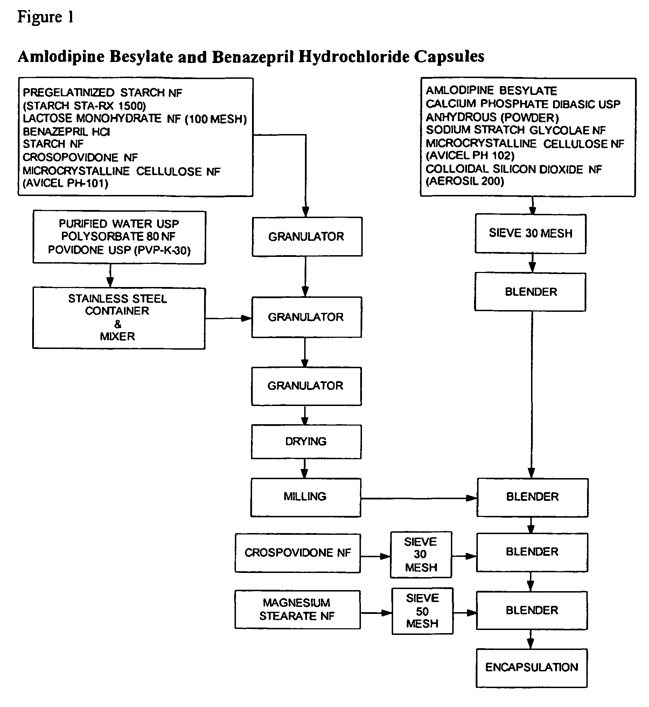

Figure 1 illustrates a flowchart for a method for making a capsule comprising a mixture of benazepril and amlodipine.

### DETAILED DESCRIPTION OF THE INVENTION

In one embodiment, the present invention provides a stable pharmaceutical composition comprising benazepril, solvates, esters, or pharmaceutically acceptable salts thereof, and amlodipine, solvates, esters, or pharmaceutically acceptable salts thereof. The general term amlodipine as used herein refers to the free base form. In the stable pharmaceutical compositions of the present invention, the benazepril is preferably benazepril hydrochloride, and the amlodipine is preferably amlodipine maleate or amlodipine besylate, more preferably amlodipine besylate.

The pharmaceutical composition is stable, that is, the pharmaceutical composition contains low levels of degradation products. In part, the composition slows and/or avoids the degradation of the active ingredient, thereby reducing the amounts of impurities present in the active ingredient after storage. A main degradation product of benazepril is (s,s)-diacid (benazeprilat), which is the active metabolite of benazepril. A main degradation product of amlodipine is impurity D (ethyl 5-methyl 2-[(2-aminoethoxy)methyl]-4-(2-chlorophenyl)-6-methylpyridine-3,5-dicarboxylate). The specifications for degradation products of benazepril and amlodipine are summarized in Table 1.

**Table 1: Limits for Degradation Products of Benazepril and Amlodipine**

| **Benazepril HCl** | |
|---|---|
| Limits are by weight percent relative to the labeled amount of benazepril HCl. | |
| (S,S)-diacid | no more than 2.5% |
| Any other impurity | no more than 0.2% |
| Total other impurities | no more than 1.0% |
| (excluding (s,s)-diacid) | |

| **Amlodipine** | |
|---|---|
| Limits are by weight percent relative to the labeled amount of amlodipine. | |
| Impurity D | no more than 0.3% |
| Any other impurity | no more than 0.2% |
| Total other impurities | no more than 1.0% |
| (excluding Impurity D) | |

In one embodiment, after the pharmaceutical composition is stored for about 3 months at about 40°C at about 75% relative humidity, the pharmaceutical composition contains no more than 2.5% of (s,s)-diacid by weight relative to the benazepril and/or no more than 0.3% of impurity D by weight relative to the amlodipine.

In a preferred embodiment, the benazepril and the amlodipine are in physical contact with one another. Examples of physical contact include physical contact at an uncoated interface between tablet layers, physical contact within the fluid medium of a liquid oral or injectable dosage form, and physical contact throughout a blended mixture of the two active ingredients. In a preferred embodiment, a blended mixture comprising the two active ingredients is used as a capsule filling.

The pharmaceutical composition preferably contains about 10 mg to about 20 mg benazepril hydrochloride, more preferably about 10 mg or about 20 mg. Preferably, the amount of amlodipine besylate corresponds to about 2.5 mg to about 10 mg of amlodipine free base. More preferably, the amount of amlodipine besylate corresponds to about 2.5 mg, about 5 mg, or about 10 mg of amlodipine free base. Preferably, the ratio of benazepril to amlodipine corresponds to a weight ratio of about 2:1 to about 4:1, more preferably about 2:1 or about 4:1.

In one embodiment, the benazepril hydrochloride is prepared by wet granulation, and the amlodipine besylate is prepared by dry processing. Wet granulation and dry processing are described in further detail below.

In one embodiment, the present invention provides a method for making a pharmaceutical composition comprising both benazepril and amlodipine. The pharmaceutical composition comprising both benazepril and amlodipine is referred to as the combination pharmaceutical composition. The method includes the steps of: preparing a benazepril composition by wet granulation; preparing an amlodipine composition by dry processing; and contacting the benazepril composition and the amlodipine composition to obtain a combination pharmaceutical composition. Fig. 1 depicts a preferred embodiment of this method.

Wet granulation preferably includes the steps of combining benazepril hydrochloride with one or more pharmaceutical excipients; adding a granulation solution to obtain a wet granulate, wherein the granulation solution preferably comprises water and optionally, one or more additional excipients; drying the wet granulate to obtain a dry granulate; and milling the dry granulate. Pharmaceutical excipients are described in further detail below. For the benazepril composition, preferred pharmaceutical excipients include pregelatinized starch, lactose monohydrate, starch, crospovidone, microcrystalline cellulose, polysorbate 80, and povidone.

Dry processing preferably includes the steps of sieving a mixture of amlodipine besylate with one or more pharmaceutical excipients; and blending the mixture. If necessary, the sieving step is performed to avoid the inclusion of agglomerates and extraneous matter. For the amlodipine composition, preferred pharmaceutical excipients include calcium phosphate dibasic, sodium starch glycolate, microcrystalline cellulose, and colloidal silicon dioxide. Preferably, the mixture is sieved through a size 30 mesh.

In one embodiment, dry processing further includes sieving one or more additional pharmaceutical excipients and blending the additional pharmaceutical excipient with the mixture, i.e., the amlodipine composition. In a preferred embodiment, the additional pharmaceutical excipient(s) are blended with the amlodipine composition after it is combined with the benazepril composition. Preferred additional pharmaceutical excipients include crospovidone and magnesium stearate. Preferably, crospovidone is sieved through a size 30 mesh. Preferably, magnesium stearate is sieved through a size 50 mesh.

In one embodiment, the method includes a step of encapsulating the combination pharmaceutical composition.

In a preferred embodiment, the method includes the steps of combining benazepril hydrochloride with one or more pharmaceutical excipients selected from the group consisting of pregelatinized starch, lactose monohydrate, starch, crospovidone, and microcrystalline cellulose; adding a granulation solution to obtain a wet granulate, wherein the granulation solution comprises water, polysorbate 80, and povidone; drying the wet granulate to obtain a dry granulate; milling the dry granulate; sieving a mixture of amlodipine besylate and one or more pharmaceutical excipients selected from the group consisting of calcium phosphate dibasic, sodium starch glycolate, microcrystalline cellulose, and colloidal silicon dioxide through a 30 mesh sieve; blending the mixture with the dry granulate to form a combined pharmaceutical composition; sieving crospovidone through a 30 mesh sieve; blending the crospovidone with the combined pharmaceutical composition; sieving magnesium stearate through a 50 mesh sieve; blending the magnesium stearate with the combined pharmaceutical composition; and encapsulating the combined pharmaceutical composition.

The compositions of the present invention can be prepared as medicaments to be administered e.g. orally. Suitable forms for oral administration include solid forms such as tablets, powders, granulates, capsules.

In addition to the active ingredient(s), the compositions of the present invention can contain one or more excipients or adjuvants. An excipient is an inert ingredient added to a pharmaceutical composition to dilute it or to give it form or consistency. An adjuvant assists the action of an active ingredient. Selection of excipients and adjuvants and the amounts to use can be readily determined by the formulation scientist based upon experience and consideration of standard procedures and reference works in the field.

Diluents increase the bulk of a solid pharmaceutical composition, and can make a pharmaceutical dosage form containing the composition easier for the patient and care giver to handle. Diluents for solid compositions include, for example, microcrystalline cellulose (e.g. Avicel^{®}), microfine cellulose, lactose, starch, pregelatinized starch, calcium carbonate, calcium sulfate, sugar, dibasic calcium phosphate dihydrate, tribasic calcium phosphate, mannitol, powdered cellulose and sorbitol.

Solid pharmaceutical compositions that are compacted into a dosage form, such as a tablet, can include excipients whose functions include helping to bind the active ingredient and other excipients together after compression. Binders for solid pharmaceutical compositions include carboxymethylcellulose sodium, ethyl cellulose, gelatin, hydroxyethyl cellulose, hydroxypropyl cellulose (e.g. Klucel^{®}), hydroxypropyl methyl cellulose (e.g. Methocel^{®}), methylcellulose, povidone (e.g. Kollidon^{®}, Plasdone^{®}), pregelatinized starch, and starch.

The dissolution rate of a compacted solid pharmaceutical composition in the patient's stomach can be increased by the addition of a disintegrant to the composition. Disintegrants include carboxymethylcellulose calcium, croscarmellose sodium (e.g. Ac-Di-Sol^{®}), crospovidone (e.g. Kollidon^{®}, Polyplasdone^{®}), microcrystalline cellulose, polacrilin potassium, pregelatinized starch, sodium starch glycolate (e.g. Explotab^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch and talc.

When a dosage form such as a tablet is made by the compaction of a powdered composition, the composition is subjected to pressure, e.g., from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc, and zinc stearate.

The dosage form of the present invention can be a capsule containing the composition, preferably a powdered and/or granulated solid composition of the invention, within either a hard or soft shell. The shell can be made from gelatin and optionally contain a plasticizer such as glycerin and sorbitol, and an opacifying agent or colorant.

Although the most suitable administration in any given case will depend on the nature and severity of the condition being treated, the most preferred route of the present invention is oral. The dosages can be conveniently presented in unit dosage form and prepared by any of the methods well-known in the pharmaceutical arts.

A composition for tableting or capsule filling can be prepared by wet granulation. In wet granulation, some or all of the active ingredients and excipients in powder form are blended and then further mixed in the presence of a liquid, typically water that causes the powders to clump into granules. The granulate is screened and/or milled, dried, screened and/or milled to the desired particle size. The granulate can then be filled into capsules optionally with other ingredients. This process is the preferred process for the Benazepril component of the present invention.

A granular composition can be prepared conventionally by dry granulation. For example, the blended composition of the actives and excipients can be compacted into a slug or a sheet and then comminuted into compacted granules. The compacted granules can subsequently be compressed into a tablet or filled into capsules. This technique is more suitable for the amlodipine component of the instant invention but even more preferred is the use of a blended powder composition of the amlodipine and excipients.

A capsule filling of the present invention can comprise any of the aforementioned blends and granulates that were described above, however, most preferred is a capsule containing a benazepril granulate prepared by wet granulation, mixed with a dry blended powder composition of the amlodipine and excipients.

Having thus described the present invention with reference to certain preferred embodiments, the invention will now be further illustrated by the following non-limiting examples.

### EXAMPLES

### Example 1: Amlodipine Besylate/Benazepril Hydrochloride Capsules

Capsules containing both benazepril hydrochloride and amlodipine besylate were made by the following process.

### Preparation of Benazepril Hydrochloride granulate - Part I:

The following materials were mixed in a granulator mixer: pregelatinized starch (Starch STA-RX-1500), lactose monohydrate NF (100 mesh), benazepril hydrochloride, starch NF, crospovidone NF, and microcrystalline cellulose NF (Avicel PH 101). The granulation solution was prepared by adding purified water USP to a mixture of polysorbate 80 NF and PVP K-30 (povidone USP) while mixing and continuing to mix until dissolved. The granulation solution was then added to the granulator mixer and mixed until a granulate was obtained. The granulate was then dried in a fluid bed drier. The dried granulate was then milled in an oscillating granulator and placed in a container.

### Dry Mix (Amlodipine) - Part II:

The following materials were sieved: amlodipine besylate, calcium phosphate dibasic USP anhydrous (powder) and sodium starch glycolate NF. The sieved materials were then transferred to a blender and blended. Microcrystalline cellulose and colloidal silicon dioxide were sieved, added to the mixture in the blender, and mixed. The mixture was then removed to a container.

### Combination of Amlodipine and Benazepril Hydrochloride - Part III:

The granulate from part I and the dry mixture from part II were placed in a blender and blended. Crospovidone was sieved and added to the blender and the mixture blended. Magnesium stearate was then sieved and added to the blender and the mixture was blended.

### Encapsulation - Part IV:

The final blend of part III was filled into capsules. Specific compositions are described in Table 2.

**Table 2: Amlodipine Besylate/Benazepril Hydrochloride Capsules**

| Ingredient | Amlodipine/Benazepril (mg) per capsule | | | |
|---|---|---|---|---|
| | 2.5 mg/10 mg | 5 mg/10 mg | 5 mg/20 mg | 10 mg/20 mg |
| **Benazepril granulate:** | 130.5 | 130.5 | 130.5 | 130.5 |
| Benazepril HCl | 10.0 | 10.0 | 20.0 | 20.0 |
| Pregelatinized Starch NF | 24.0 | 24.0 | 22.0 | 22.0 |
| (Starch STA-RX 1500) | | | | |
| Lactose Monohydrate NF | 39.2 | 39.2 | 35.2 | 35.2 |
| (100 mesh) | | | | |
| Starch NF | 16.0 | 16.0 | 15.0 | 15.0 |
| Crospovidone NF | 7.0 | 7.0 | 7.0 | 7.0 |
| Microcrystalline Cellulose NF (Avicel PH-101) | 27.0 | 27.0 | 24.0 | 24.0 |
| **Granulation solvent:** | | | | |
| Polysorbate 80 NF | 0.8 | 0.8 | 0.8 | 0.8 |
| Povidone USP (PVP K-30) | 6.5 | 6.5 | 6.5 | 6.5 |
| Purified Water USP | * | * | * | * |
| **Amlodipine composition:** | 139.5 | 139.5 | 139.5 | 139.5 |
| Amlodipine Besylate | 3.465** | 6.93** | 6.93** | 13.86** |
| Calcium Phosphate Dibasic USP Anhydrous (powder) | 40.0 | 39.0 | 39.0 | 37.0 |
| Sodium Starch Glycolate NF | 2.0 | 2.0 | 2.0 | 2.0 |
| Microcrystalline Cellulose NF | 83.135 | 80.67 | 80.67 | 75.74 |
| (Avicel PH-102) | | | | |
| Colloidal Silicon Dioxide NF | 1.4 | 1.4 | 1.4 | 1.4 |
| (Aerosil 200) | | | | |
| **Added after combining with benazepril composition:** | | | | |
| Crospovidone NF | 7.0 | 7.0 | 7.0 | 7.0 |
| (30 mesh) | | | | |
| Magnesium Stearate NF | 2.5 | 2.5 | 2.5 | 2.5 |
| (50 mesh) | | | | |
| **Total** | **270.0** | **270.0** | **270.0** | **270.0** |

| | | | | |
|---|---|---|---|---|
| * For granulation processing only. ** 3.465 mg, 6.93 mg, and 13.86 mg of amlodipine besylate correspond to 2.5 mg, 5 mg, and 10 mg of amlodipine free base, respectively. | | | | |

### Example 2: Stability Study

Over the course of 3 months at 40°C and 75% relative humidity, the degradation products of the amlodipine besylate/benazepril hydrochloride capsules were measured by HPLC. Results are summarized in Table 3 below.

**Table 3: Stability Results for Amlodipine Besylate/Benazepril Hydrochloride Capsules**

| | | Time zero | 1M | | | 2M | | | 3M | | |
|---|---|---|---|---|---|---|---|---|---|---|---|
| | | | | 40c/75%RH | | 40c/75%RH | | 40c/75%RH | | PP CAP | PP CAP |
| 2.5/10mg | *s,s-diacid | | 100 Tabs | 100 Tabs | 1000 Tabs | 100 Tabs | 100 Tabs | 1000 Tabs | 100 Tabs | 100 Tabs | 1000 Tabs |
| | Total(excl *) | | CRC¹ | PP CAP² | PP CAP | CRC | PP CAP | PP CAP | CRC | PP CAP | PP CAP |
| | | | | | | | | | | | |
| | **Impurity D | <0.05 | 0.08 | - | - | 0.06 | - | - | 0.1 | - | - |
| | Total(excl *) | <0.05 | <0.05 | - | - | <0.05 | - | - | <0.05 | - | - |
| 5/10mg | *s,s-diacid | <0.05 | 0.3 | 0.4 | 0.4 | 0.6 | 1.1 | 0.8 | 1.0 | 1.2 | 1.2 |
| | Total(excl *) | 0.07 | 0.1 | 0.1 | 0.1 | 0.2 | 0.2 | 0.2 | 0.2 | 0.2 | 0.2 |
| | | | | | | | | | | | |
| | **Impurity D | <0.05 | 0.1 | 0.1 | 0.1 | 0.08 | 0.1 | 0.08 | 0.1 | 0.1 | 0.1 |
| | Total(excl *) | <0.05 | <0.05 | <0.05 | <0.05 | <0.05 | <0.05 | <0.05 | <0.05 | <0.05 | <0.05 |
| 5/20mg | *s,s-diacid | <0.05 | 0.2 | 0.3 | 0.2 | 0.4 | 0.6 | 0.4 | 0.8 | 1.0 | 0.8 |
| | Total(excl *) | 0.06 | 0.05 | 0.06 | 0.05 | 0.1 | 0.1 | 0.1 | 0.1 | 0.1 | 0.1 |
| | | | | | | | | | | | |
| | **Impurity D | <0.05 | 0.1 | 0.1 | 0.1 | 0.06 | 0.08 | 0.07 | 0.1 | 0.09 | 0.1 |
| | Total(excl *) | <0.05 | <0.05 | <0.05 | <0.05 | <0.05 | <0.05 | <0.05 | 0.06 | 0.07 | 0.08 |
| 10/20mg | *s,s-diacid | <0.05 | 0.2 | - | - | 0.3 | - | - | 0.6 | - | - |
| | Total(excl *) | 0.07 | 0.1 | - | - | 0.1 | - | - | 0.2 | - | - |
| | | | | | | | | | | | |
| | **Impurity D | <0.05 | 0.06 | - | - | 0.06 | - | - | 0.1 | - | - |
| | Total(excl *) | <0.05 | <0.05 | - | - | <0.05 | - | - | <0.05 | - | - |

| | | | | | | | | | | | |
|---|---|---|---|---|---|---|---|---|---|---|---|
| *s,s-diacid results from degradation of Benazepril **Impurity D results from degradation of Amlodipine ¹CRC = Child resistant Cap ²PP CAP = Polypropylene Cap | | | | | | | | | | | |

## Claims

1. A pharmaceutical composition comprising:
a) Benazepril or its solvates, or a pharmaceutically acceptable salt thereof, and
b) Amlodipine or its solvates, or a pharmaceutically acceptable salt thereof, such that the benazepril and the amlodipine are in physical contact with one another, and wherein after 3 months at 40°C at 75% relative humidity, the pharmaceutical composition contains:
i) no more than 2.5% of (s,s)-diacid by weight relative to the benazepril; and/or
ii) no more than 0.3% of impurity D by weight relative to the amlodipine.

2. The pharmaceutical composition of claim 1, wherein the pharmaceutical composition is encapsulated.

3. The pharmaceutical composition of any preceding claim, wherein the benazepril is benazepril hydrochloride.

4. The pharmaceutical composition of any preceding claim, wherein the amlodipine is amlodipine besylate.

5. The pharmaceutical composition of any preceding claim, wherein the benazepril is prepared by wet granulation, and the amlodipine is prepared by dry processing.

6. A method comprising:
a) preparing a benazepril composition by wet granulation;
b) preparing an amlodipine composition by dry processing; and
c) contacting the benazepril composition and the amlodipine composition, and optionally adding excipients, to obtain a combination pharmaceutical composition.

7. The method of claim 6, wherein the benazepril composition comprises benazepril hydrochloride and one or more pharmaceutical excipients.

8. The method of claim 6 or claim 7, wherein benazepril composition comprises one or more pharmaceutical excipients selected from the group consisting of pregelatinized starch, lactose monohydrate, starch, crospovidone, microcrystalline cellulose, polysorbate 80, and povidone.

9. The method of any of claims 6 to 8, wherein the benazepril composition comprises:
a) 5% to 20% benazepril hydrochloride;
b) 15% to 20% pregelatinized starch;
c) 25% to 35% lactose monohydrate;
d) 10% to 15% starch;
e) 5% crospovidone;
f) 15% to 25% microcrystalline cellulose;
g) 0.1 % to 2% polysorbate 80; and
h) 5% povidone,
by weight percent relative to the total benazepril composition.

10. The method of any of claims 6 to 9, wherein preparing the benazepril composition by wet granulation comprises:
a) combining benazepril hydrochloride with one or more pharmaceutical excipients selected from the group consisting of pregelatinized starch, lactose monohydrate, starch, crospovidone, and microcrystalline cellulose;
b) adding a granulation solution to obtain a wet granulate, wherein the granulation solution comprises water and optionally, polysorbate 80, povidone, or both;
c) drying the wet granulate to obtain a dry granulate; and
d) milling the dry granulate.

11. The method of any of claims 6 to 10, wherein the amlodipine composition comprises amlodipine besylate and one or more pharmaceutical excipients.

12. The method of any of claims 6 to 11, wherein the amlodipine composition comprises one or more pharmaceutical excipients selected from the group consisting of calcium phosphate dibasic, sodium starch glycolate, microcrystalline cellulose, colloidal silicon dioxide.

13. The method of any of claims 6 to 12, wherein the amlodipine composition comprises:
a) 2% to 10% amlodipine besylate;
b) 25% to 30% calcium phosphate dibasic;
c) 1% to 2% sodium starch glycolate;
d) 50% to 60% microcrystalline cellulose;
e) 1% colloidal silicon dioxide,
by weight percent relative to the total amlodipine composition.

14. The method of claim 6, wherein the optionally added excipients comprise crospovidone and magnesium stearate.

15. The method of claim 14, wherein the optionally added excipients comprise 1% to 5% crospovidone and 0.2% to 2% magnesium stearate by weight percent relative to the total amlodipine composition.

16. The method of any of claims 6 to 15, wherein preparing the amlodipine composition by dry processing comprises:
a) sieving a mixture of amlodipine besylate and one or more pharmaceutical excipients selected from the group consisting of calcium phosphate dibasic, sodium starch glycolate, microcrystalline cellulose, and colloidal silicon dioxide; and
b) blending the mixture.

17. The method of any of claims 6 to 16, further comprising:
a) sieving one or more additional pharmaceutical excipients selected from the group consisting of crospovidone and magnesium stearate; and
b) blending the additional pharmaceutical excipient with the mixture.

18. The method of any of claims 6 to 17, further comprising encapsulating the combined pharmaceutical composition.

19. The method of any of claims 6 to 18, comprising:
a) combining benazepril hydrochloride with one or more pharmaceutical excipients selected from the group consisting of pregelatinized starch, lactose monohydrate, starch, crospovidone, and microcrystalline cellulose;
b) adding a granulation solution to obtain a wet granulate, wherein the granulation solution comprises water, polysorbate 80, and povidone;
c) drying the wet granulate to obtain a dry granulate;
d) milling the dry granulate;
e) sieving a mixture of amlodipine besylate and one or more pharmaceutical excipients selected from the group consisting of calcium phosphate dibasic, sodium starch glycolate, microcrystalline cellulose, and colloidal silicon dioxide, through a 30 mesh sieve;
f) blending the mixture with the dry granulate to form a combined pharmaceutical composition;
g) sieving crospovidone through a 30 mesh sieve;
h) blending the crospovidone with the combined pharmaceutical composition;
i) sieving magnesium stearate through a 50 mesh sieve;
j) blending the magnesium stearate with the combined pharmaceutical composition; and
k) encapsulating the combined pharmaceutical composition.

## Patentansprüche

1. Pharmazeutische Zusammensetzung, welche folgendes umfaßt:
a) Benazepril oder dessen Solvate oder ein pharmazeutisch verträgliches Salz davon und
b) Amlodipin oder dessen Solvate oder ein pharmazeutisch verträgliches Salz davon, so daß das Benazepril und das Amlodipin in physischem Kontakt miteinander sind, und wobei nach 3 Monaten bei 40°C und 75% relativer Feuchte die pharmazeutische Zusammensetzung folgendes enthält.
i) nicht mehr als 2,5 Gewichts-% (s,s)-Disäure relativ zu dem Benazepril und/oder
ii) nicht mehr als 0,3 Gewichts-% Verunreinigung D relativ zu dem Amlodipin.

2. Pharmazeutische Zusammensetzung nach Anspruch 1, wobei die pharmazeutische Zusammensetzung eingekapselt ist.

3. Pharmazeutische Zusammensetzung nach einem der vorangegangenen Ansprüche, wobei das Benazepril Benazeprilhydrochlorid ist.

4. Pharmazeutische Zusammensetzung nach einem der vorangegangenen Ansprüche, wobei das Amlodipin Amlodipinbesylat ist.

5. Pharmazeutische Zusammensetzung nach einem der vorangegangenen Ansprüche, wobei das Benazepril durch Naßgranulation hergestellt wird und das Amlodipin durch Trockenverarbeitung hergestellt wird.

6. Verfahren, welches folgendes umfaßt:
a) Herstellen einer Benazepril-Zusammensetzung mittels Naßgranulation,
b) Herstellen einer Amlodipin-Zusammensetzung mittels Trockenverarbeitung und
c) Inkontaktbringen der Benazepril-Zusammensetzung und der Amlodipin-Zusammensetzung und optional Zugeben von Hilfsstoffen unter Erhalt einer kombinierten pharmazeutischen Zusammensetzung.

7. Verfahren nach Anspruch 6, wobei die Benazepril-Zusammensetzung Benazeprilhydrochlorid und einen oder mehrere pharmazeutische Hilfsstoffe umfaßt.

8. Verfahren nach Anspruch 6 oder Anspruch 7, wobei die Benazepril-Zusammensetzung einen oder mehrere pharmazeutische Hilfsstoffe umfaßt, ausgewählt aus der Gruppe, bestehend aus vorgelatinierter Stärke, Lactosemonohydrat, Stärke, Crospovidon, mikrokristalliner Zellulose, Polysorbat 80 und Povidon.

9. Verfahren nach einem der Ansprüche 6 bis 8, wobei die Benazepril-Zusammensetzung folgendes umfaßt:
a) 5% bis 20% Benazeprilhydrochlorid,
b) 15% bis 20% vorgelatinierte Stärke,
c) 25% bis 35% Lactosemonohydrat,
d) 10% bis 15% Stärke,
e) 5% Crospovidon,
f) 15% bis 25% mikrokristalline Zellulose,
g) 0,1% bis 2% Polysorbat 80 und
h) 5% Povidon
in Gewichtsprozent relativ zu der Benazepril-Zusammensetzung insgesamt.

10. Verfahren nach einem der Ansprüche 6 bis 9, wobei die Herstellung der Benazepril-Zusammensetzung mittels Naßgranulation folgendes umfaßt:
a) Vereinigen von Benazeprilhydrochlorid mit einem oder mehreren pharmazeutischen Hilfsstoffen, ausgewählt aus der Gruppe, bestehend aus vorgelatinierter Stärke, Lactosemonohydrat, Stärke, Crospovidon und mikrokristalliner Zellulose,
b) Zugeben einer Granulationslösung unter Erhalt eines nassen Granulats, wobei die Granulationslösung Wasser und optional Polysorbat 80, Povidon oder beides enthält,
c) Trocknen des nassen Granulats unter Erhalt eines trockenen Granulats und
d) Mahlen des trockenen Granulats.

11. Verfahren nach einem der Ansprüche 6 bis 10, wobei die Amlodipin-Zusammensetzung Amlodipinbesylat und einen oder mehrere pharmazeutische Hilfsstoffe umfaßt.

12. Verfahren nach einem der Ansprüche 6 bis 11, wobei die Amlodipin-Zusammensetzung einen oder mehrere pharmazeutische Hilfsstoffe umfaßt, ausgewählt aus der Gruppe, bestehend aus zweibasigem Calciumphosphat, Natriumstärkeglycolat, mikrokristalliner Zellulose, kolloidalem Siliciumdioxid.

13. Verfahren nach einem der Ansprüche 6 bis 12, wobei die Amlodipin-Zusammensetzung folgendes umfaßt:
a) 2% bis 10% Amlodipinbesylat,
b) 25% bis 30% zweibasiges Calciumphosphat,
c) 1% bis 2% Natriumstärkeglycolat,
d) 50% bis 60% mikrokristalline Zellulose,
e) 1% kolloidales Siliciumdioxid
in Gewichtsprozent relativ zu der Amlodipin-Zusammensetzung insgesamt.

14. Verfahren nach Anspruch 6, wobei die optional zugegebenen Hilfsstoffe Crospovidon und Magnesiumstearat umfassen.

15. Verfahren nach Anspruch 14, wobei die optional zugegebenen Hilfsstoffe in Gewichtsprozent 1% bis 5% Crospovidon und 0,2% bis 2% Magnesiumstearat relativ zu der Amlodipin-Zusammensetzung insgesamt umfassen.

16. Verfahren nach einem der Ansprüche 6 bis 15, wobei die Herstellung der Amlodipin-Zusammensetzung mittels Trockenverarbeitung folgendes umfaßt:
a) Sieben eines Gemischs von Amlodipinbesylat und einem oder mehreren pharmazeutischen Hilfsstoffen, ausgewählt aus der Gruppe, bestehend aus zweibasigem Calciumphosphat, Natriumstärkeglycolat, mikrokristalliner Zellulose und kolloidalem Siliciumdioxid, und
b) Vermischen des Gemischs.

17. Verfahren nach einem der Ansprüche 6 bis 16, welches weiterhin folgendes umfaßt:
a) Sieben eines oder mehrerer zusätzlicher pharmazeutischer Hilfsstoffe, ausgewählt aus der Gruppe, bestehend aus Crospovidon und Magnesiumstearat, und
b) Vermischen des zusätzlichen pharmazeutischen Hilfsstoffs mit dem Gemisch.

18. Verfahren nach einem der Ansprüche 6 bis 17, welches weiterhin das Einkapseln der kombinierten pharmazeutischen Zusammensetzung umfaßt.

19. Verfahren nach einem der Ansprüche 6 bis 18, welches folgendes umfaßt:
a) Vereinigen von Benazeprilhydrochlorid mit einem oder mehreren pharmazeutischen Hilfsstoffen, ausgewählt aus der Gruppe, bestehend aus vorgelatinierter Stärke, Lactosemonohydrat, Stärke, Crospovidon und mikrokristalliner Zellulose,
b) Zugeben einer Granulationslösung unter Erhalt eines nassen Granulats, wobei die Granulationslösung Wasser, Polysorbat 80 und Povidon umfaßt,
c) Trocknen des nassen Granulats unter Erhalt eines trockenen Granulats,
d) Mahlen des trockenen Granulats,
e) Sieben eines Gemischs aus Amlodipinbesylat und einem oder mehreren pharmazeutischen Hilfsstoffen, ausgewählt aus der Gruppe, bestehend aus zweibasigem Calciumphosphat, Natriumstärkeglycolat, mikrokristalliner Zellulose und kolloidalem Siliciumdioxid, durch ein 30-Maschen-Sieb,
f) Vermischen des Gemischs mit dem trockenen Granulat unter Bildung einer kombinierten pharmazeutischen Zusammensetzung,
g) Sieben von Crospovidon durch ein 30-Maschen-Sieb,
h) Vermischen des Crospovidons mit der kombinierten pharmazeutischen Zusammensetzung,
i) Sieben von Magnesiumstearat durch ein 50-Maschen-Sieb,
j) Vermischen des Magnesiumstearats mit der kombinierten pharmazeutischen Zusammensetzung und
k) Einkapseln der kombinierten pharmazeutischen Zusammensetzung.

## Revendications

1. Composition pharmaceutique comprenant :
a) le benazepril ou ses solvates, ou un de ses sels acceptables sur le plan pharmaceutique, et
b) l'amlodipine ou ses solvates, ou un de ses sels acceptables sur le plan pharmaceutique, de sorte que le benazepril et l'amlodipine sont en contact physique l'un avec l'autre, et dans laquelle après 3 mois à 40°C et à une humidité relative de 75%, la composition pharmaceutique ne contient :
i) pas plus de 2,5% de (s,s)-diacide en poids par rapport au benazepril ; et/ou
ii) pas plus de 0,3% d'impureté D en poids par rapport à l'amlodipine.

2. Composition pharmaceutique selon la revendication 1, dans laquelle la composition pharmaceutique est encapsulée.

3. Composition pharmaceutique selon l'une quelconque des revendications précédentes, dans laquelle le benazepril est le chlorhydrate de benazepril.

4. Composition pharmaceutique selon l'une quelconque des revendications précédentes, dans laquelle l'amlodipine est le bésylate d'amlodipine.

5. Composition pharmaceutique selon l'une quelconque des revendications précédentes, dans laquelle le benazepril est préparé par granulation humide, et l'amlodipine est préparée selon un procédé par voie sèche.

6. Procédé comprenant les étapes consistant à :
a) préparer une composition de benazepril par granulation humide ;
b) préparer une composition d'amlodipine selon un procédé par voie sèche ; et
c) mettre en contact la composition de benazepril et la composition d'amlodipine, et ajouter éventuellement des excipients pour obtenir une composition pharmaceutique en combinaison.

7. Procédé selon la revendication 6, dans lequel la composition de benazepril comprend le chlorhydrate de benazepril et un ou plusieurs excipients pharmaceutiques.

8. Procédé selon la revendication 6 ou 7, dans lequel la composition de benazepril comprend un ou plusieurs excipients pharmaceutiques choisis dans le groupe constitué par l'amidon prégélifié, le lactose monohydraté, l'amidon, la crospovidone, la cellulose microcristalline, le polysorbate 80 et la povidone.

9. Procédé selon l'une quelconque des revendications 6 à 8, dans lequel la composition de benazepril comprend :
a) 5% à 20% de chlorhydrate de benazepril ;
b) 15% à 20% d'amidon prégélifié ;
c) 25% à 35% de lactose monohydraté ;
d) 10% à 15% d'amidon ;
e) 5% de crospovidone ;
f) 15% à 25% de cellulose microcristalline ;
g) 0,1 % à 2% de polysorbate 80 ; et
h) 5% de povidone,
en pourcentage pondéral par rapport à la composition totale de benazepril.

10. Procédé selon l'une quelconque des revendications 6 à 9, dans lequel la préparation de la composition de benazepril par granulation humide comprend les étapes consistant à :
a) combiner le chlorhydrate de benazepril avec un ou plusieurs excipients pharmaceutiques choisis dans le groupe constitué par l'amidon prégélifié, le lactose monohydraté, l'amidon, la crospovidone et la cellulose microcristalline ;
b) ajouter une solution de granulation pour obtenir un granulé humide, dans lequel la solution de granulation comprend de l'eau et éventuellement, le polysorbate 80, la povidone ou les deux ;
c) sécher le granulé humide pour obtenir un granulé sec ; et
d) broyer le granulé sec.

11. Procédé selon l'une quelconque des revendications 6 à 10, dans lequel la composition d'amlodipine comprend le bésylate d'amlodipine et un ou plusieurs excipients pharmaceutiques.

12. Procédé selon l'une quelconque des revendications 6 à 11, dans lequel la composition d'amlodipine comprend un ou plusieurs excipients pharmaceutiques choisis dans le groupe constitué par le phosphate de calcium dibasique, le glycolate d'amidon sodique, la cellulose microcristalline, le dioxyde de silicium colloïdal.

13. Procédé selon l'une quelconque des revendications 6 à 12, dans lequel la composition d'amlodipine comprend :
a) 2% à 10% de bésylate d'amlodipine ;
b) 25% à 30% de phosphate de calcium dibasique ;
c) 1% à 2% de glycolate d'amidon sodique ;
d) 50% à 60% de cellulose microcristalline ;
e) 1% de dioxyde de silicium colloïdal,
en pourcentage pondéral par rapport à la composition totale d'amlodipine.

14. Procédé selon la revendication 6, dans lequel les excipients éventuellement ajoutés comprennent la crospovidone et le stéarate de magnésium.

15. Procédé selon la revendication 14, dans lequel les excipients éventuellement ajoutés comprennent 1% à 5% de crospovidone et 0,2% à 2% de stéarate de magnésium en pourcentage pondéral par rapport à la composition totale d'amlodipine.

16. Procédé selon l'une quelconque des revendications 6 à 15, dans lequel la préparation de la composition d'amlodipine selon un procédé par voie sèche comprend les étapes consistant à :
a) tamiser un mélange de bésylate d'amlodipine et un ou plusieurs excipients pharmaceutiques choisis dans le groupe constitué par le phosphate de calcium dibasique, le glycolate d'amidon sodique, la cellulose microcristalline et le dioxyde de silicium colloïdal ; et
b) mélanger le mélange.

17. Procédé selon l'une quelconque des revendications 6 à 16, comprenant en outre les étapes consistant à :
a) tamiser un ou plusieurs excipients pharmaceutiques supplémentaires choisis dans le groupe constitué par la crospovidone et le stéarate de magnésium ; et
b) mélanger l'excipient pharmaceutique supplémentaire avec le mélange.

18. Procédé selon l'une quelconque des revendications 6 à 17, comprenant en outre l'étape consistant à encapsuler la composition pharmaceutique combinée.

19. Procédé selon l'une quelconque des revendications 6 à 18, comprenant les étapes consistant à :
a) combiner le chlorhydrate de benazepril avec un ou plusieurs excipients pharmaceutiques choisis dans le groupe constitué par l'amidon prégélifié, le lactose monohydraté, l'amidon, la crospovidone et la cellulose microcristalline ;
b) ajouter une solution de granulation pour obtenir un granulé humide, dans lequel la solution de granulation comprend de l'eau, le polysorbate 80 et la povidone ;
c) sécher le granulé humide pour obtenir un granulé sec ;
d) broyer le granulé sec ;
e) tamiser un mélange de bésylate d'amlodipine et un ou plusieurs excipients pharmaceutiques choisis dans le groupe constitué par le phosphate de calcium dibasique, le glycolate d'amidon sodique, la cellulose microcristalline et le dioxyde de silicium colloïdal à travers un tamis à ouverture de maille de 0,6 mm (30 mesh) ;
f) mélanger le mélange avec le granulé sec pour former une composition pharmaceutique combinée ;
g) tamiser la crospovidone à travers un tamis à ouverture de maille de 0,6 mm (30 mesh) ;
h) mélanger la crospovidone avec la composition pharmaceutique combinée ;
i) tamiser le stéarate de magnésium à travers un tamis à ouverture de maille de 0,3 mm (50 mesh) ;
j) mélanger le stéarate de magnésium avec la composition pharmaceutique combinée ; et
k) encapsuler la composition pharmaceutique combinée.
